# EUROPÄISCHE PATENTSCHRIFT

(11) **EP 0 472 143 B1**
(45) Veröffentlichungstag und Bekanntmachung des Hinweises auf die Patenterteilung: **05.04.1995**
(21) Anmeldenummer: 91113869.1
(22) Anmeldetag: 19.08.1991
(51) Int. Cl.: C07C 227/40, C07F 9/30, C07C 229/24

(54) **Verfahren zur extraktiven Trennung von L-Phosphinothricin und L-Glutaminsäure in wässriger Lösung**
Process for the extractive separation of 1-phosphinothricin and l-glutamic acid in aqueous solution
Procédé pour la séparation extractive de l-phosphinothricine d'acide l-glutamique en solution aqueuse

(30) Priorität: 23.08.1990 DE 4026628
(43) Veröffentlichungstag der Anmeldung: 26.02.1992
(73) Patentinhaber: Hoechst Schering AgrEvo GmbH, 13342 Berlin (DE)
(72) Erfinder: Mildenberger, Hilmar, Dr., W-6233 Kelkheim/Ts. (DE); Knorr, Harald, Dr., W-6000 Frankfurt a.M. (DE); Schulz, Eckhard, Dr., W-6239 Eppstein/Ts. (DE); Tetzlaff, Heribert, Dr., D-63128 Dietzenbach . (DE)

(56) Entgegenhaltungen:
- EP-A- 0 248 357
- EP-A- 0 344 683
- FR-A- 251 852
- NATURE, Band 177, 1956, London, GB; PAR-AKE ALBERTSSON: "Chromatography and partition of cells and cell fragments", Seiten 771-774

## Beschreibung

Zur Trennung von Aminosäuren sind verschiedene Verfahren gebräuchlich. Dazu zählen z.B. die Elektrodialyse, (Ionenaustausch)chromatographie-, Extraktions- und Kristallisationsverfahren. Ein Großteil dieser Verfahren versagt jedoch oder ist mit erheblichen Nachteilen verbunden, wenn sehr polare, hydrophile Aminosäuren mit nahe beieinanderliegenden isoelektrischen Punkten (IEP) aufgetrennt werden sollen. Eine solche Kombination stellt die Mischung aus L-Glutaminsäure (IEP = 3,24) und L-Phosphinothricin (IEP = 2,6) dar. Solche Mischungen fallen z.B. bei der Darstellung des L-Phosphinothricins (L-PTC, d. h. L-2-Amino-4-(hydroxymethylphosphinyl)-butansäure) durch enzymatische Transaminierung von α-Ketosäure (d. h. 4-(Hydroxymethylphosphinyl)-2-oxo-butansäure) in Gegenwart eines Überschusses an L-Glutaminsäure an (s. z. B. EP-A-248357).

Das Verteilungsverhalten der beiden Aminosäuren bei der Flüssig-Flüssig-Extraktion (d.h. bei ihrer Verteilung zwischen zwei nicht mischbaren Phasen) wird durch ihre Verteilungskoeffizienten bestimmt, die als das Verhältnis der Aminosäurekonzentration in einer Phase zur Konzentration in der anderen Phase definiert sind. Der Quotient aus den Verteilungskoeffizienten der beiden Aminosäuren heißt Trennfaktor und ist ein Maß für den Trennaufwand (Stufenzahl, Phasenmengenverhältnis), der bei einer Gegenstromverteilung betrieben werden muß, um die beiden Aminosäuren bis zu einem bestimmten Grad voneinander zu trennen. Je größer der Trennfaktor, desto geringer ist die benötigte theoretische Stufenzahl.

In EP-A-0247436, EP-A-0166992 und RO-114808 werden Extraktionsverfahren beschrieben, bei denen mit Hilfe von wasserunlöslichen Phasentransferkatalysatoren (flüssigen Ionenaustauschern) wie quarternären Stickstoff-, Schwefel-, Phosphor- oder Borbasen oder auch mit wasserunlöslichen Sulfonsäuren die Aminosäuren Lysin, Phenylalanin, Tryptophan und Glutaminsäure aus wäßriger Lösung abgetrennt werden können. Prinzipiell ist auch L-PTC mit diesen Extraktionsmitteln aus wäßriger Lösung extrahierbar, jedoch liegen die Verteilungskoeffizienten für die beiden polaren, hydrophilen Aminosäuren Glutaminsäure und L-PTC in den betreffenden Zweiphasensystemen jeweils nur im engen Bereich von 0,1 bis 0,2. Sie können daher nach den angegebenen Methoden nicht wirtschaftlich voneinander getrennt werden (s. unten Vergleichsbeispiele).

In DE-A-1277256 ist ein Verfahren beschrieben, bei dem Aminosäuren mit Hilfe von Dinonylnaphthalinsulfonsäure oder deren Natriumsalz als Kationenaustauscher und Dichlormethan aus wäßriger Lösung von pH 2-5 extrahiert werden. Aber die Extrahierbarkeit von L-PTC und L-Glutaminsäure ist für ein wirtschaftlich anzuwendendes Verfahren zu gering, weil zu hohe Mengen an Extraktionsphase benötigt werden. Zudem ist die Aufarbeitung der Extrakte durch die dort beschriebene Verdrängungsextraktion mit organischen Aminen zu umständlich.

In US-A-4,536,596 wird ein Verfahren beschrieben, bei dem mit Hilfe von Dithiophosphinaten Aminosäuren, die nur eine Carboxylgruppe enthalten, aus wäßriger Lösung extrahiert werden. Allerdings ist das Verfahren nicht zur Extraktion von L-Glutaminsäure geeignet (s. US-A-4,536,596, Sp. 7, Tab. III), und somit auch nicht zur Extraktion des noch hydrophileren L-PTC.

In JP-14444/68 wird ein Verfahren beschrieben, bei dem Aminosäuren, wie unter anderem auch Glutaminsäure, mit Tri-(niedr.alkyl)-phosphaten aus wäßriger Lösung extrahiert werden können. Die Methode ist jedoch zur Trennung von L-Glutaminsäure und L-PTC nicht effektiv genug (s. unten Vergleichsbeispiele).

In DE-A-2822870 werden Aminosäuren aus wäßriger Lösung mit einer Mischung aus einem aliphatischem C₄-C₈-Keton und teilneutralisierter Cumolsulfonsäure extrahiert. Dazu müssen die Aminosäuren in der hydrogensulfatsauren Form vorliegen. Bei diesem Verfahren wird eine große Anzahl von Prozeßschritten benötigt und eine große Menge an Salzen als Abfall gebildet, so daß das Verfahren für eine technische Anwendung weniger geeignet ist.

In EP-A-0251852 wird ein Verfahren beschrieben, bei dem mit Hilfe von Dialkyl/arylphosphorsäuren, -phosphonsäuren oder -phosphinsäuren Aminosäuren aus wäßriger Lösung extrahiert werden können. L-Glutaminsäure ist jedoch nicht genügend mit Organophosphorsäuren extrahierbar (s. unten Vergleichsbeispiele).

Wegen der o.g. Nachteile ist keines der erwähnten Verfahren für eine effektive Trennung von L-Glutaminsäure und L-PTC aus wäßriger Lösung geeignet.

Gegenstand der Erfindung ist ein Verfahren zur Trennung von L-Phosphinothricin und L-Glutaminsäure, dadurch gekennzeichnet, daß die Trennung als Flüssig-Flüssig-Extraktion mit zwei nicht mischbaren wäßrigen Phasen als Phasensystem durchgeführt wird, dabei als Phasenbildner im Phasensystem eine Kombination von mindestens zwei unterschiedlichen wasserlöslichen Polymeren oder von mindestens einem wasserlöslichen Salz und einem wasserlöslichen Polymeren enthalten ist und die Extraktion mehrstufig im Gegenstrom nach dem Prinzip der van Dijck-Verteilung durchgeführt wird.

Die für das erfindungsgemäße Verfahren anwendbaren Phasensysteme aus nicht mischbaren wäßrigen Phasen sind im Prinzip seit langem bekannt (s. z.B. P.-A. Albertsson, Nature, Vol. 177, 771 (1956) und P.-A. Albertsson in "Partition of Cell Particles and Macromolecules", 2nd Edition/3rd Edition, John Wiley & Sons, New York 1971 bzw. 1986). Man erzeugt sie beispielsweise durch Zumischen zweier unterschiedlicher, wasserlöslicher Polymere oder von einem Polymer und einem Salz zu einer wäßrigen Phase. Bisher war bekannt, daß sich bei der Verteilung von Stoffen zwischen zwei derartigen wäßrigen Phasen nur für große Moleküle (z.B. DNA) Verteilungskoeffizienten über 100, für kleinere wie Proteine und Enzyme bis zu 10 erreichen lassen, während sich kleine Moleküle und Salze in der Regel etwa gleich verteilen (s. z.B. P.-A. Albertsson, Nature, Vol. 177 (1956) 773 ff.). Überraschenderweise ergibt sich jedoch auch für die relativ kleinen Moleküle L-Glutaminsäure und L-PTC ein ausreichender bis guter Trennfaktor, wenn das genannte Phasensystem aus zwei nicht mischbaren wäßrigen Phasen eingesetzt wird.

Geeignete Polymer-Polymer-Kombinationen als Phasenbildner für das erfindungsgemäße Verfahren sind beispielsweise:
a1) Polyethylenglykol oder Polyethylenglykolmonoalkylether oder -dialkylether in Kombination mit Polyvinylalkohol, Polyvinylpyrrolidon, Dextran oder synthetische Polysaccharide wie ®Ficoll (Molekulargewicht ca. 400000);
a2) Polyvinylalkohol in Kombination mit Methylcellulose oder Dextran;
a3) Polyvinylpyrrolidon in Kombination mit Dextran;
a4) synthetische Polysaccharide wie ®Ficoll in Kombination mit Dextran.

Geeignete Polymer-Salz-Kombinationen sind beispielsweise:
b1) Polyethylenglykol oder Polyethylenglykolmonoalkylether oder -dialkylether in Kombination mit Kaliumsulfat, Kaliumphosphat oder Ammoniumsulfat oder auch mit Na-Dextransulfat oder Na-Carboxymethylcellulose;
b2) Polyvinylpyrrolidon in Kombination mit Kaliumphosphat;
b3) Polypropylenglykol in Kombination mit Glucose;
b4) Polyvinylalkohol in Kombination mit Na-Dextransulfat oder Na-Carboxymethylcellulose.

Als Zweiphasenbildner sind die vorstehend unter der Gruppe b1) genannten von speziellem Interesse. Dabei sind Polyethylenglykoldialkylether, vorzugsweise Polyethylenglykoldimethylether mit Molekulargewichten zwischen 250 und 2000, insbesondere 400 bis 800, in Kombination mit Ammoniumsulfat hervorzuheben. Das Ammoniumsulfat wird vorzugsweise im Konzentrationsbereich von 10 bis 40 Gew.-%, bezogen auf die das Salz enthaltende Phase, eingesetzt.

Das Gewichtsverhältnis der beiden nicht mischbaren Phasen beträgt vorzugsweise 1:10 bis 10:1. Der Trennfaktor ist abhängig vom pH-Wert des Phasensystems und ist in der Regel im pH-Bereich von 0 bis 12,5. Bevorzugt ist eine Arbeitsweise bei pH 2,8 bis 3,3, insbesondere pH 3. Die Temperatur wird bei dem erfindungsgemäßen Verfahren zweckmäßig so eingestellt, daß ein möglichst guter Trennfaktor bei gleichzeitig guter Phasentrennung erreicht wird. Sie ist in der Regel im Bereich von 10 bis 60 °C und vorzugsweise bei Raumtemperatur.

Das zu trennende Gemisch von L-PTC und L-Glutaminsäure wird in der Regel als wäßrige Losung dem Zweiphasensystem zugeführt, wobei die wäßrige Lösung noch andere Bestandteile enthalten kann. Beispielsweise kann eine wäßrige Lösung des zu trennenden Gemisches zugeführt werden, wie sie direkt nach der enzymatischen Transaminierung von α-Ketosäure in Gegenwart eines Überschusses an L-Glutaminsäure anfällt.

Das Extraktionsschema nach der van Dijck-Verteilung ist bekannt; siehe z. B. O. Jübermann in Houben Weyl Bd. I/1a, G. Thieme Verlag, Stuttgart 1958, Seiten 249 bis 255.
Dabei wird beispielsweise die Lösung des Gemisches mit L-Glutaminsäure und L-PTC in die Mitte eines mehrstufigen Extraktor-Verteilungsbetts aus zwei nicht mischbaren wäßrigen und im Gegenstrom geführten Phasen einspeist. Durch die mehrstufige Arbeitsweise nach Art der van Dijck-Verteilung gelingt es, an einem Ende des Verteilungsbetts eine wäßrige Lösung zu erhalten, die praktisch nur noch L-PTC und den einen Teil der Phasenbildner-Kombination, z. B. Ammoniumsulfat, enthält, während am anderen Ende eine wäßrige Lösung anfällt, die praktisch nur noch L-Glutaminsäure und den zweiten Teil der Phasenbildner-Kombination, z. B. Polyethylenglykoldimethylether, enthält.

In einer bevorzugten Ausführungsform des Verfahrens wird beispielsweise die wäßrige Lösung eines Gemisches, das 1 bis 20 Gew.-%, vorzugsweise 1 bis 15 Gew.-% L-Glutaminsäure und 1 bis 15 Gew.-%, vorzugsweise 1 bis 10 Gew.-% L-PTC bei Raumtemperatur und pH 3 in der Mitte des Extraktionsapparats in das Verteilungsbett aus den beiden nicht mischbaren wäßrigen Phasen eingepumpt. Der pH-Wert dieser Phasen ist zuvor ebenfalls auf 3 eingestellt worden. Es wird Polyethylenglykoldimethylether mit einem Molekulargewicht von 500 (PEGE 500) und Ammoniumsulfat als 2 Phasen bildendes System eingesetzt. Die beiden nicht mischbaren wäßrigen Phasen werden zuvor durch Herstellung einer Lösung oder Dispersion aus 21 Gew.-% Ammoniumsulfat und 26 Gew.-% PEGE 500 erzeugt, die spontan etwa hälftig in die beiden wäßrigen Phasen zerfällt. Das in der Mitte zugegebene Aminosäuregemisch wird dann mehrstufig, im Gegenstrom, zwischen den beiden wäßrigen Phasen verteilt. Aus der an einem Ende des Extraktionsapparats ablaufenden Oberphase, die die L-Glutaminsäure und PEGE 500 enthält, wird nach Neutralisation das Wasser destillativ entfernt und die daraufhin ausfallende, im PEGE 500 unlösliche L-Glutaminsäure durch Filtrieren abgetrennt. PEGE 500 wird in die Extraktion (über die Stufe der 2-Phasen-Bildung) zurückgeführt. Aus der am anderen Ende der Apparatur ablaufenden Unterphase wird nach Neutralisation das Wasser destillativ bis auf 6 bis 8 Gew.-% entfernt, dann durch Methanolzugabe das Ammoniumsulfat ausgefällt. Nach dem Abfiltrieren des Salzes (das ebenfalls zurückgeführt wird) wird das Methanol abgedampft und die verbleibende Schmelze kristallisiert oder als anwendungsfertige wäßrige Lösung von L-PTC eingestellt.

Für die extraktive Trennung von L-Glutaminsäure und L-PTC nach dem Schema der van Dijck-Verteilung sind als Apparate prinzipiell alle gängigen Extraktionsapparate geeignet (Kolonnen, Mischabsetzer, Graesser-Extraktor, Zentrifugalextraktor), jedoch ist wegen der benötigten Stufenzahlen der Einsatz von Mischabsetzerbatterien oder Extraktionskolonnen bevorzugt.

Das Verfahren wird anhand der folgenden Beispiele und Vergleichsbeispiele erläutert:

### Beispiel 1

Die folgende Tabelle 1 zeigt die Verteilung von L-Glutaminsäure und L-PTC zwischen nicht mischbaren wäßrigen Phasen, gebildet aus Polyethylenglykoldimethylethern verschiedener Kettenlänge und Ammoniumsulfat bei Raumtemperatur. Anhand der angegebenen Trennfaktoren (β) wird besonders die Abhängigkeit des Trennfaktors vom pH-Wert deutlich:

**Tabelle 1**

| pH | 0,44 | 2,07 | 3,03 | 4,02 | 8,25 | 12,35 |
|---|---|---|---|---|---|---|
| β (PEGE 500) | 1,33 | 1,15 | 1,78 | 1,34 | 1,17 | 1,54 |
| β (PEGE 1000) | 1,30 | 1,01 | 1,20 | 1,18 | 1,34 | 1,34 |
| β (PEGE 2000) | 1,31 | 1,22 | 1,37 | 1,24 | 1,22 | 1,32 |

### Beispiel 2

Eine Transaminierungslösung, erhalten nach dem Verfahren aus EP-A-248357 und auf pH 3 eingestellt, mit 4 Gew.-% L-Glutaminsäure und 1,5 Gew.-% L-PTC wurde bei Raumtemperatur in die Mitte eines 20-stufigen Labormischabsetzers eingespeist. Am einen Ende des Labormischabsetzers wurde eine wäßrige Phase, ca. 20 Gew.-% PEGE 500 enthaltend, am anderen Ende eine wäßrige Phase, ca. 25 Gew.-% Ammoniumsulfat enthaltend, eingespeist. Das Mengenverhältnis von Transaminierungslösung zu PEGE 500-Phase bzw. Ammoniumsulfatphase betrug 1:3:1. Die am einen Ende der Apparatur ablaufende PEGE 500-Phase enthielt ca. 95 %-ige L-Glutaminsäure, die am anderen Ende ablaufende Ammoniumsulfatphase enthielt ca. 95 %-iges L-PTC. Aus der PEGE 500-Phase wurde die Glutaminsäure durch Abdampfen des Wassers und Filtration gewonnen, aus der Ammoniumsulfatphase wurde das Mineralsalz nach Neutralisation und Eindampfen durch Methanolzugabe abgeschieden.

### Beispiel 3

Eine auf pH 7,9 eingestellte Transaminierungslösung mit 13,1 Gew.-% L-Glutaminsäure und 3,9 Gew.-% L-PTC wurde bei Raumtemperatur in die Mitte einer Schwingbodenkolonne (Länge 6 m, Durchmesser 50 mm, Typ Karr) eingespeist. Am unteren Ende der Kolonne wurde eine wäßrige Phase, ca. 20 Gew.-% PEGE 500 enthaltend, als disperse Phase eingespeist. Am oberen Ende wurde eine wäßrige Phase, ca. 25 Gew.-% Ammoniumsulfat enthaltend, als kontinuierliche Phase eingespeist. Das Mengenverhältnis Transaminierungslösung : PEGE 500-Phase : Ammoniumsulfatphase betrug 0,7 : 3,2 : 1,1. Die am oberen Ende der Kolonne ablaufende PEGE 500-Phase enthielt ca. 96 %-ige L-Glutaminsäure, die am unteren Ende ablaufende Ammoniumsulfatphase enthielt ca. 94 %-iges L-PTC. Aus der PEGE 500-Phase wurde die L-Glutaminsäure durch Abdampfen des Wassers und Filtration gewonnen, aus der Ammoniumsulfatphase wurde das Mineralsalz nach Neutralisation und Eindampfen durch Methanolzugabe abgeschieden.

### Vergleichsbeispiele

Die Tabelle 2 zeigt anhand eines Vergleichs des Verhältnisses der Konzentration der L-Glutaminsäure zur Konzentration des L-PTC vor und nach der Extraktion, daß eine Auftrennung der beiden Aminosäuren mit Hilfe von oben zum Stand der Technik genannten Extraktionsmitteln nicht wirtschaftlich ist, da die allenfalls geringen Änderungen im Konzentrationsverhältnis eine Trennung in einer Gegenstromextraktion mit vertretbarer Stufenzahl nicht zulassen.

**Tabelle 2**

| Extraktionsmittel | Konzentrationsverhältnis L-Glu/L-PTC bei pH | | | | |
|---|---|---|---|---|---|
| | 1,8 | 4,8 | 8,9 | 11,0 | 12,0 |
| Keines (Anfangswert) | 4,5 | 4,8 | 4,9 | 4,8 | 4,8 |
| BDOPO | 4,7 | 4,9 | 4,9 | - | - |
| ®Hostarex A327 | - | 4,9 | 4,9 | - | 4,9 |
| ®Hostarex-chlorid | 4,7 | - | - | - | - |
| Nonylphenol | 5,2 | 4,6 | 4,7 | - | - |
| Isononansäure | 5,0 | 4,9 | 4,9 | - | - |
| Isobutylisooctylamin | - | 4,9 | 4,9 | - | - |
| Di-n-octylamin | - | 4,8 | 4,8 | - | - |
| ®Hostarex DK16 | 4,9 | 4,9 | 4,9 | - | - |
| Phosphorsäure-di-(2-ethylhexyl)-ester | 4,8 | - | 4,7 | 4,9 | - |
| Tributylphosphat | - | - | 4,8 | - | 4,9 |
| Tn-OABr in ®Solvesso 100 | - | - | 4,7 | 4,9 | 4,9 |
| 50 Gew.-% Dinonylnaphthalinsulfonsäure/Kerosin | 4,8 | 4,9 | - | - | - |
| Abkürzungen: BDOPO = 2-Butyl)-di-(n-octyl)-phosphanoxid ®Hostarex A327 = Tri-(n-octyl)-amin : Tri-(n-decyl)-amin (1 : 1) ®Hostarex DK16 = Alkyliertes Benzoylaceton Tn-OABr = Tetra-(n-Octyl)-ammoniumbromid ®Solvesso 100 = Aromatengemisch mit 80 Gew.-% meta-C₉-Aromaten | | | | | |

## Patentansprüche

1. Verfahren zur Trennung von L-Phosphinothricin und L-Glutaminsäure, dadurch gekennzeichnet, daß die Trennung als Flüssig-Flüssig-Extraktion mit zwei nicht mischbaren wäßrigen Phasen als Phasensystem durchgeführt wird, dabei als Phasenbildner im Phasensystem eine Kombination von mindestens zwei unterschiedlichen wasserlöslichen Polymeren oder von mindestens einem wasserlöslichen Salz und einem wasserlöslichen Polymeren enthalten ist und die Extraktion mehrstufig im Gegenstrom nach dem Prinzip der van Dijck-Verteilung durchgeführt wird.

2. Verfahren nach Anspruch 1, dadurch gekennzeichnet, daß als Zweiphasenbildner Polyethylenglykol oder Polyethylenglykolmonoalkylether oder -dialkylether in Kombination mit Kaliumsulfat, Kaliumphosphat oder Ammoniumsulfat eingesetzt werden.

3. Verfahren nach Anspruch 1 oder 2, dadurch gekennzeichnet, daß als Phasenbildner Polyethylenglykoldimethylether mit Molekulargewichten zwischen 250 und 2000 in Kombination mit Ammoniumsulfat eingesetzt werden.

4. Verfahren nach Anspruch 2 oder 3, dadurch gekennzeichnet, daß Ammoniumsulfat in
einer Konzentration von 10 bis 40 Gew.-%, bezogen auf die das Salz enthaltende Phase, eingesetzt wird.

5. Verfahren nach einem oder mehreren der Ansprüche 1 bis 4, dadurch gekennzeichnet, daß der pH-Wert des Phasensystems 0 bis 12,5 beträgt.

6. Verfahren nach Anspruch 5, dadurch gekennzeichnet, daß der pH-Wert 2,8 bis 3,3 beträgt.

7. Verfahren nach einem oder mehreren der Ansprüche 1 bis 6, dadurch gekennzeichnet, daß das Gewichtsverhältnis der beiden nicht mischbaren Phasen 1:10 bis 10:1 beträgt.

8. Verfahren nach einem oder mehreren der Ansprüche 1 bis 7, dadurch gekennzeichnet, daß die Trennung bei 10 bis 60 °C durchgeführt wird.

9. Verfahren nach einem oder mehreren der Ansprüche 1 bis 8, dadurch gekennzeichnet, daß das zu trennende Gemisch als wäßrige Lösung eingesetzt wird, wie sie nach einer enzymatischen Transaminierung von 4-(Hydroxymethylphosphinyl)-2-oxo-butansäure in Gegenwart eines Überschusses an L-Glutaminsäure erhalten wird.

10. Verfahren nach einem oder mehreren der Ansprüche 1 bis 9, dadurch gekennzeichnet, daß das zu trennende Gemisch 1 bis 20 Gew.-% L-Glutaminsäure und 1 bis 15 Gew.-% L-PTC enthält.

11. Verfahren nach Anspruch 10, dadurch gekennzeichnet, daß das zu trennende Gemisch 1 bis 15 Gew.-% L-Glutaminsäure und 1 bis 10 Gew.-% L-PTC enthält.

12. Verfahren nach einem oder mehreren der Ansprüch 1 bis 11, dadurch gekennzeichnet, daß die Aufarbeitung der das Verteilungsbett verlassenden Phasen destillativ erfolgt.

## Claims

1. A process for separating L-phosphinothricin and L-glutamic acid, which comprises carrying out the separation as liquid/liquid extraction with two non-miscible aqueous phases as phase system, where the phase former contained in the phase system is a combination of at least two different water-soluble polymers or of at least one water-soluble salt and one water-soluble polymer, and the extraction is carried out by multistage counter-current partition on the van Dijck principle.

2. The process as claimed in claim 1, wherein polyethylene glycol or polyethylene glycol monoalkyl ether or dialkyl ether in combination with potassium sulfate, potassium phosphate or ammonium sulfate are employed as two-phase former.

3. The process as claimed in claim 1 or 2, wherein polyethylene glycol dimethyl ether with molecular weights between 250 and 2000 in combination with ammonium sulfate are employed as phase former.

4. The process as claimed in claim 2 or 3, wherein ammonium sulfate in a concentration of 10 to 40% by weight, based on the salt-containing phase, is employed.

5. The process as claimed in one or more of claims 1 to 4, wherein the pH of the phase system is 0 to 12.5.

6. The process as claimed in claim 5, wherein the pH is 2.8 to 3.3.

7. The process as claimed in one or more of claims 1 to 6, wherein the ratio by weight of the two non-miscible phases is 1:10 to 10:1.

8. The process as claimed in one or more of claims 1 to 7, wherein the separation is carried out at 10 to 60°C.

9. The process as claimed in one or more of claims 1 to 8, wherein the mixture to be separated is employed as aqueous solution as obtained after enzymatic transamination of 4-(hydroxymethylphosphinyl)-2-oxobutanoic acid in the presence of an excess of L-glutamic acid.

10. The process as claimed in one or more of claims 1 to 9, wherein the mixture to be separated contains 1 to 20% by weight L-glutamic acid and 1 to 15% by weight L-PTC.

11. The process as claimed in claim 10, wherein the mixture to be separated contains 1 to 15% by weight L-glutamic acid and 1 to 10% by weight L-PTC.

12. The process as claimed in one or more of claims 1 to 11, wherein the phases leaving the partition bed are worked up by distillation.

## Revendications

1. Procédé pour séparer la L-phosphinothricine et l'acide L-glutamique, procédé caractérisé en ce qu'on effectue la séparation sous forme d'une extraction liquide/liquide avec deux phases aqueuses non miscibles servant de système de phases, le système des phases comportant comme générateur des phases une combinaison d'au moins deux polymères hydrosolubles différents ou d'au moins un sel hydrosoluble et un polymère hydrosoluble, et l'extraction étant réalisée en plusieurs étapes, en opérant à contre-courant selon le principe de la répartition de partage de Dijck.

2. Procédé selon la revendication 1, caractérisé en ce qu'on utilise, comme générateur des deux phases, du polyéthylène glycol ou un éther monoalkylique ou un éther dialkylique de polyéthylène glycol en combinaison avec du sulfate de potassium, du phosphate de potassium ou du sulfate d'ammonium.

3. Procédé selon la revendication 1 ou 2 caractérisé en ce qu'on utilise comme générateur des phases de l'éther diméthylique de polyéthylène glycol ayant un poids moléculaire compris entre 250 et 2000, en combinaison avec du sulfate d'ammonium.

4. Procédé selon la revendication 2 ou 3, caractérisé en ce que l'on utilise le sulfate d'ammonium en une concentration de 10 à 40 % en poids, par rapport à la phase contenant le sel.

5. Procédé selon une ou plusieurs des revendications 1 à 4, caractérisé en ce que la valeur du pH du système des phases vaut 0 à 12,5.

6. Procédé selon la revendication 5, caractérisé en ce que la valeur du pH se situe entre 2,8 et 3,3.

7. Procédé selon une ou plusieurs des revendications 1 à 6, caractérisé en ce que le rapport pondéral entre les deux phases non miscibles vaut de 1:10 à 10:1.

8. Procédé selon une ou plusieurs des revendications 1 à 7, caractérisé en ce que l'on conduit la séparation à une température de 10 à 60°C.

9. Procédé selon une ou plusieurs des revendications 1 à 8, caractérisé en qu'on introduit le mélange à séparer sous la forme d'une solution aqueuse telle qu'on l'obtient après une transamination enzymatique de l'acide 4-(hydroxyméthylphosphinyl)-2-oxo-butanoïque en présence d'un excès d'acide L-glutamique.

10. Procédé selon une ou plusieurs des revendications 1 à 9, caractérisé en ce que le mélange à séparer contient 1 à 20 % en poids d'acide L-glutamique et 1 à 15 % en poids de L-PTC.

11. Procédé selon la revendication 10, caractérisé en ce que le mélange à séparer contient 1 à 15 % en poids d'acide L-glutamique et 1 à 10 % en poids de L-PTC.

12. Procédé selon une ou plusieurs des revendications 1 à 11, caractérisé en ce que le traitement de purification des phases sortant du lit de partage est réalisé par distillation.
